**Europäisches Patentamt**

**European Patent Office**

**Office européen des brevets**

(19)

(11) Numéro de publication : **0 309 309 B1**

(12)

# FASCICULE DE BREVET EUROPEEN

(45) Date de publication du fascicule du brevet :
**22.04.92 Bulletin 92/17**

(51) Int. Cl.⁵ : **A61K 7/48,** A61K 7/00,
A45D 44/00, A61F 13/12

(21) Numéro de dépôt : **88402258.3**

(22) Date de dépôt : **08.09.88**

(54) **Matériau en feuille pour réaliser un traitement cutané ou capillaire, son procédé de fabrication et articles réalisés en ce matériau.**

(30) Priorité : **25.09.87 FR 8713264**

(43) Date de publication de la demande :
**29.03.89 Bulletin 89/13**

(45) Mention de la délivrance du brevet :
**22.04.92 Bulletin 92/17**

(84) Etats contractants désignés :
**BE CH DE ES GB IT LI SE**

(56) Documents cités :
**EP-A- 0 063 875
WO-A-87/05206
AT-B- 206 114
FR-A- 2 276 030
FR-A- 2 503 561
US-A- 3 428 043
US-A- 4 377 160
US-A- 4 643 939**

(73) Titulaire : **L'OREAL
14, Rue Royale
F-75008 Paris (FR)**

(72) Inventeur : **Gueret, Jean-Louis
15, Rue Hégésippe-Moreau
F-75018 Paris (FR)**
Inventeur : **Contamin, Jean-Claude
14, Avenue du Château
F-91420 Morangis (FR)**
Inventeur : **Ayache, Liliane
277, Rue de Charenton
F-75012 Paris (FR)**

(74) Mandataire : **Peuscet, Jacques et al
Cabinet Peuscet 68, rue d'Hauteville
F-75010 Paris (FR)**

EP 0 309 309 B1

Jouve, 18, rue Saint-Denis, 75001 PARIS

## Description

La présente invention se rapporte à un matériau composite en feuille qui s'utilise par application temporaire sur le revêtement cutané ou sur la chevelure, pour réaliser un traitement localisé, cutané ou capillaire, notamment à effet cosmétique. L'invention se rapporte également à la fabrication de ce matériau et à son mode d'utilisation.

Selon ses caractéristiques de structure, de composition et de forme, le matériau selon l'invention peut constituer un masque destiné aux soins du visage ou une partie du visage (contour des yeux, joue, menton) afin notamment de nettoyer la peau en profondeur par élimination des couches cornées de surface, de raffermir la peau, de l'adoucir, ou de lui faire subir un traitement particulier ; le matériau selon l'invention peut également constituer un élément utile pour le raffermissement du buste, ou encore des pastilles pour soins localisés, du type pastilles anti-rides. Concernant l'application sur la chevelure, on peut mentionner l'utilisation du matériau selon l'invention dans les traitements de conditionnement capillaire et les mises en plis.

Les produits du type "masque de beauté" sont bien connus dans le domaine de la cosmétique. Ils se présentent notamment sous forme de gels ou pâtes qui, après application sur le visage, sèchent pour donner un film que l'on retire par lavage, nettoyage ou arrachage. Pendant le temps du séchage, la couche cornée, ou stratum corneum, s'humidifie et s'assouplit, et la peau se contracte légèrement. Après séchage, le flux sanguin est accéléré par suite d'une élévation de température. En outre, le masque présente un effet d'absorption, et lorsqu'il est retiré, il assure donc un nettoyage en profondeur de la peau en même temps qu'un "peeling" entraînant notamment lesdites couches cornées de surface.

On connaît par la demande de brevet européen n° 0 063 875, un masque pour le visage constitué par une feuille, qui est convenablement découpée à la forme du visage et qui porte des ingrédients cosmétiques liquides destinés à être transférés lors de l'application de la feuille sur le visage.

On connaît également par les demandes de brevet français n° 2 512 651 et n° 2 538 247, des pastilles anti-rides incorporant, dans leur matière ou dans des cavités, des substances cosmétiques ou pharmaceutiques. Là également, le produit de traitement est directement transféré sur la peau.

Le brevet autrichien n° 206 114 décrit un masque de beauté ayant une forme correspondant à l'anatomie du visage qui est constitué par un tissu de soie imprégné de gel (alcool polyvinylique) et recouvert d'une couche de pâte contenant des extraits de plantes et/ou des vitamines. Le masque est constitué de deux couches, donc épais et doit être réhumidifié juste avant l'utilisation.

US-A-3 428 041 décrit un pansement constitué par une feuille sèche d'un gel hydrophile dans laquelle on a introduit une substance active en la trempant dans une solution de cette substance active. Cette feuille est renforcée par un tissu de type gaze que l'on colle sur la feuille, place entre deux feuilles ou noie dans une couche préparée avec deux feuilles. Le pansement est humidifié avant usage en le trempant dans un liquide approprié.

US-A-4 377 160 décrit un bandage constitué par une bande de mousse de matière plastique imprégnée d'un gel hydrophile d'alcool polyvinylique. le gel d'alcool polyvinilique n'est pas séché et le bandage est stocké humide puis appliqué humide sur la peau.

La présente invention concerne, entre autres, un nouveau type de masque d'emploi très commode puisque, contrairement aux gels, pâtes et feuilles précités, il peut s'appliquer à l'état sec sur le visage après humidification de celui-ci, et qu'il s'enlève tout aussi facilement, ce masque procurant dans tous les cas, comme les gels et les pâtes, une désincrustation efficace des pores de la peau. De plus, le masque selon l'invention peut contenir dans la couche de gel des substances actives permettant de réaliser un traitement complémentaire par transfert desdites substances sur la peau.

Le masque selon l'invention est constitué par un filet emprisonant un gel hydratable en couche mince dans ses mailles. Le gel est utilisé pour ses propriétés d'absorption de l'eau apportée par l'humidification de la surface de la peau : à partir de sa forme sèche légèrement rigide, il s'hydrate au moins partiellement et devient souple, le filet retrouvant alors sa flexibilité naturelle et venant épouser les aspérités du corps (creux de l'oeil, creux des rides) ; puis il sèche sous l'influence de la température normale de la peau, la forme sèche étant reprise alors qu'il adhère toujours sur la peau. Du fait que le gel sec est toujours prisonnier dans son filet, on peut alors retirer le complexe (filet/gel) d'un seul tenant, un traitement de désincrustation de la peau ayant été effectué par ledit gel.

L'utilisation d'une mince couche de gel seule en combinaison avec un filet d'armature, n'avait jamais été mise en évidence jusqu'ici. D'autre part la couche de gel avait toujours été appliquée à l'état humidifié sur la peau.

Il est, en outre, intéressant de profiter de la fonction de véhicule que possède le gel pour libérer, à l'hydratation du gel, et mettre en contact avec la peau, au moins un produit de traitement.

En outre, on peut prévoir selon l'invention, que le filet imprégné, découpé à la dimension de la partie du corps à traiter, soit appliqué sur le côté adhésif d'une feuille à revêtement adhésif, imperméable à l'eau à l'état liquide ou vapeur, en laissant en périphérie de ladite feuille, la couche adhésive non revêtue par le filet, de façon à créer une pastille, un masque ou plus généralement, un élément occlusif, lorsqu'il est collé sur la peau. Dans ce cas, l'eau initialement déposée sur la peau hydrate le gel et solubilise, le cas échéant, la substance traitante qu'il contient, ladite substance pouvant, dès lors, être libérée pour agir sur la peau. L'eau de la transpiration ne traverse pas le masque en raison de la présence de la feuille imperméable et, en se condensant à la surface de la peau, elle contribue à la dissolution du produit actif, l'effet de transpiration provoquant une ouverture des pores, qui favorise la pénétration du produit actif. On active donc ainsi le phénomène de transdermie. On peut, en variante, si la feuille-support présenté une certaine perméabilité au cours du temps, ne réaliser qu'une occlusion partielle.

La présente invention a donc d'abord pour premier objet un matériau en feuille pour réaliser un traitement cutané local ou un traitement de conditionnement capillaire, lors d'une application temporaire respectivement sur le revêtement cutané ou la chevelure, caractérisé par le fait qu'il comprend un filet flexible tissé ou non tissé, ayant une épaisseur comprise entre 0,01 et 2 mm, comptant environ 2 à 100 mailles par centimètre, le rapport de la surface de ses ouvertures à sa surface totale étant compris entre 5 et 80 %, filet qui constitue l'armature d'une couche de gel hydratable ayant une épaisseur moyenne comprise entre 5 et 1000 μm.

On préfère que le filet soit réalisé en une matière hydrophobe, c'est-à-dire non absorbante. On le choisit avantageusement en une matière synthétique comme le polyamide, de préférence au coton, auquel il est toutefois possible de faire appel. On préfère également que le filet soit en matière transparente car il est ainsi possible, le cas échéant, de voir quand l'application est terminée en observant par transparence, par exemple une modification de couleur au cours du traitement. De plus les masques transparents sont plus esthétiques. On utilise également de préférence un filet en substance pouvant être stérilisée à haute température. L'utilisation de filets en polyamide convient particulièrement. En effet ils permettent de déposer une quantité de gel plus faible et parfaitement contrôlée par l'épaisseur de la trame et des interstices formés par les mailles du filet.

Le filet peut être constitué d'un tissu tissé ou non-tissé ayant une épaisseur comprise entre 0,01 et 2 mm, comptant environ 2 à 100 mailles par centimètre, le rapport de la surface de ses ouvertures à sa surface totale étant compris notamment entre 5 et 80%. On utilise de préférence un filet en tissu tissé car souvent les tissus non tissés contiennent des liants qui peuvent favoriser la prolifération de microorganismes au cours du stockage ou lors de la réhydratation au cours de l'application. D'autre part les filets tissés épousent mieux les différentes formes du corps que les non-tissés.

Egalement, on utilise, de préférence, un filet extensible dans une seule direction, la raison de ce choix tenant à la fabrication de l'article selon l'invention ; en effet, comme on le décrit ci-après, dans une fabrication industrielle, le filet doit pouvoir être bobiné et débobiné sans problème ; un filet extensible dans les deux directions s'étirerait au bobinage.

Le gel est avantageusement constitué d'un polymère hydrosoluble ou hydrogonflable, présentant un extrait sec compris entre 0,5 et 50% en poids. Les polymères que l'on peut utiliser à cet effet sont avantageusement pris dans le groupe formé par l'alcool polyvinylique, les sels de métaux alcalins de carboxyméthylcellulose réticulée, les sels de métaux alcalins d'acide polyacrylique, les poly(oxyde d'alkylène) réticulés, les sels de métaux alcalins de polymères greffés cellulose-acrylonitrile ou amidonacrylonitrile contenant des groupements carboxyliques, la gomme adragante, la gomme arabique, la gomme de guar et ses dérivés, les alginates, la gomme de xanthane, les autres dérivés de la cellulose, l'albumine, la gélatine, le galactomannane, le polyacrylamide.

Le filet, entre les mailles duquel est emprisonné le gel, permet d'utiliser une faible épaisseur de gel ; sans cette armature le gel, pris dans cette faible épaisseur, constituerait une feuille qui n'aurait pas suffisamment de tenue pour pouvoir être manipulée. Or, une faible épaisseur de gel est nécessaire au moment de l'application du masque (ou analogue) pour que ce dernier puisse absorber rapidement l'humidité de la partie traitée humidifiée pour assurer l'assouplissement rapide du gel et donc du complexe (filet/gel) ; pour qu'en conséquence, le masque (ou analogue) puisse épouser les formes souvent sinueuses des parties traitées du visage ou du corps ; pour qu'enfin il y ait une évaporation rapide de l'eau réabsorbée, afin d'assécher de nouveau le gel, dans le cas où le masque n'est pas occlusif. L'épaisseur moyenne du gel sur le filet sera généralement comprise entre 5 et 1000 μm.

Par ailleurs, le gel peut avantageusement, renfermer au moins une substance active de traitement, notamment à des fins cosmétiques, choisie notamment parmi les substances suivantes : des actifs hydrosolubles, l'argile, le kaolin, la silice, les huiles émulsionnées, les agents hydratants, amincissants, anti-rides, stimulants, revitalisants, raffermissants, et adoucissants, les poudres et similaires. Dans ce cas il est particulièrement important que l'épaisseur moyenne du gel sur le filet soit faible. En effet, lorsque l'épaisseur de la couche de gel est trop grande, ladite couche est très difficile à humecter convenablement et, même si le masque devient suffisamment souple, la couche non réhydratée absorbe l'eau et empêche les substances actives de rester en contact avec la partie du corps traitée.

Lorsque la masque comporte au moins deux substances actives, on peut prévoir lesdites substances actives soient localisées en des emplacements différents sur le filet. Cette possibilité sera utilisée avantageusement lorsque les produits actifs ne sont pas compatibles entre eux. Dans ce cas on peut également humidifier, juste avant application par exemple à l'aide d'un flacon muni d'un tampon, la surface de gel qui sera appliqué sur la peau, avec une solution aqueuse ou hydroalcoolique contenant les substances actives non compatibles avec celles contenues dans le gel sec.

Le matériau selon l'invention peut servir à constituer un article, qui présente avantageusement une découpe adaptée à la forme de la partie du corps à traiter.

On peut notamment prévoir dans le cas d'un masque destiné au traitement du visage, que ledit masque soit réalisé en deux parties, l'une correspondant au front et se prolongeant, le long de sa bordure inférieure, entre deux échancrures qui suivent le contour des arcades sourcillières, par une patte destinée à recouvrir le haut du nez, et l'autre, correspondant à la région du visage se situant au-dessous des yeux et comportant deux ouvertures correspondant respectivement à la bouche et au nez, la bordure supérieure de cette partie du masque comportant deux échancrures pour suivre la ligne inférieure des yeux. Ces masques en deux parties s'ajustent bien aux différentes formes de visages.

Suivant une autre possibilité de réalisation d'un masque destiné au traitement du visage, celui-ci est constitué d'une seule pièce découpée à la forme et à la dimension du visage, ladite pièce comprenant des ouvertures correspondant respectivement aux yeux et à la bouche, ainsi qu'une découpe correspondant à la région médiane et inférieure du nez et dégageant un volet destiné à venir recouvrir le nez, une fente médiane reliant l'ouverture correspondant à la bouche et ladite découpe, des fentes transversales étant également pratiquées le long des bordures latérales de ladite pièce.

Egalement, selon l'invention, on peut prévoir des éléments pour le raffermissement du buste, comprenant une découpe sensiblement en forme de demi-couronne.

Selon l'invention, le filet imprégné peut être supporté, sur l'une de ses faces, par une feuille-support protectrice détachable, et le cas échéant, protégé sur son autre face, par une autre feuille protectrice détachable. Dans le cas des masques totalement ou partiellement occlusifs, le filet imprégné par le gel, découpé à la dimension correspondant à la partie du corps à traiter, est disposé sur le côté adhésif d'une feuille-support (imperméable ou présentant une perméabilité partielle si le masque est partiellement occlusif) revêtue, sur l'une de ses faces, d'une douche d'adhésif, ladite feuille dépassant dudit filet imprégné de gel sur tout son pourtour, une pellicule détachable venant protéger au moins la partie adhésive avant emploi. On peut également prévoir que plusieurs éléments de filet imprégné comportant chacun une substance active différente soient disposés séparément sur une même feuille-support.

Par ailleurs, que le masque selon l'invention soit ou non occlusif, on peut prévoir qu'au moins une zone du filet imprégnée par le gel soit rendue imperméable partiellement ou totalement grâce à une fraction de feuille-support de surface limitée.

Le matériau selon l'invention pourrait également être associé à un matériau spongieux saturé d'au moins une substance de traitement non compatible dans le temps avec le gel et/ou avec la (ou les) substance(s) de traitement contenue(s), le cas échéant, dans le gel.

La présente invention concerne également un procédé de fabrication d'un matériau selon l'invention comportant une feuille-support, ce procédé étant caractérisé par le fait que :
– on place une nappe constituant un filet flexible sur une feuille-support capable de résister à la température de chauffage du gel hydraté à l'étape ultérieure de séchage;
– on dépose sur ladite nappe ainsi supportée, une mince couche d'un gel hydraté se trouvant dans un état liquide ou extrudable et renfermant, le cas échéant, au moins une substance active de traitement, de façon à charger les mailles du filet constitutif de la nappe avec ledit gel hydraté ;
– on sèche ledit gel hydraté jusqu'à atteindre le degré de déshydratation désiré.

Pour obtenir ensuite un article réalisé au moyen du matériau ainsi fabriqué, on découpe ledit matériau de façon appropriée et on le conditionne pour l'utilisation prévue.

Le couchage du gel hydraté peut s'effectuer par calandrage ou par extrusion.

On peut effectuer le séchage du gel hydraté prisonnier du filet par calandrage ou en faisant passer ledit filet dans un tunnel infra-rouge ou en soufflant de l'air chaud sur celui-ci, une pellicule de protection étant placée par dessus ledit filet supporté dans le cas où l'on procède par calandrage.

L'invention concerne également l'utilisation du matériau ci-dessus défini pour un traitement cosmétique cutané ou un conditionnement capillaire, selon laquelle, après avoir humidifié la partie du corps à traiter ou la chevelure, on y applique le filet imprégné du gel hydratable, solidaire de sa feuille-support dans le cas où l'on réalise un traitement au moins partiellement occlusif ; on maintient l'application pendant un temps suffisant pour assurer le séchage du gel qui s'est réhydraté au contact de la région humidifiée, et on retire le filet par arrachage d'un seul tenant.

Pour mieux faire comprendre, l'objet de la présente invention, on va décrire ci-après une forme possible de mise en oeuvre du procédé de fabrication d'un filet imprégné de gel selon l'invention, ainsi qu'à titre d'exemples purement illustratifs et non limitatifs, plusieurs modes de réalisation d'articles constitués avec ledit filet imprégné, l'ensemble étant représenté sur le dessin annexé.

Sur ce dessin :

– la figure 1 est un schéma d'ensemble illustrant une forme de mise en oeuvre du procédé selon l'invention ;

– les figures 2A et 2B sont des vues en coupe, à échelle agrandie, du matériau composite obtenu par la mise en oeuvre du procédé schématisé sur la figure 1, ces deux matériaux différant l'un de l'autre selon la quantité de gel appliqué sur le filet ;

– la figure 3 est une vue de dessus d'un masque pour le visage, réalisé en deux parties, qui a été découpé dans un matériau composite selon l'invention ;

– la figure 4 est une vue analogue à la figure 3 d'un masque réalisé en une seule pièce ;

– la figure 5 est une vue de dessus d'un article selon l'invention destiné au raffermissement du buste ;

– la figure 6 est une vue schématique illustrant la réalisation d'une pastille occlusive avec un matériau de l'invention ; et

– la figure 7 est une vue schématique d'une variante de réalisation de la pastille occlusive de la figure 6.

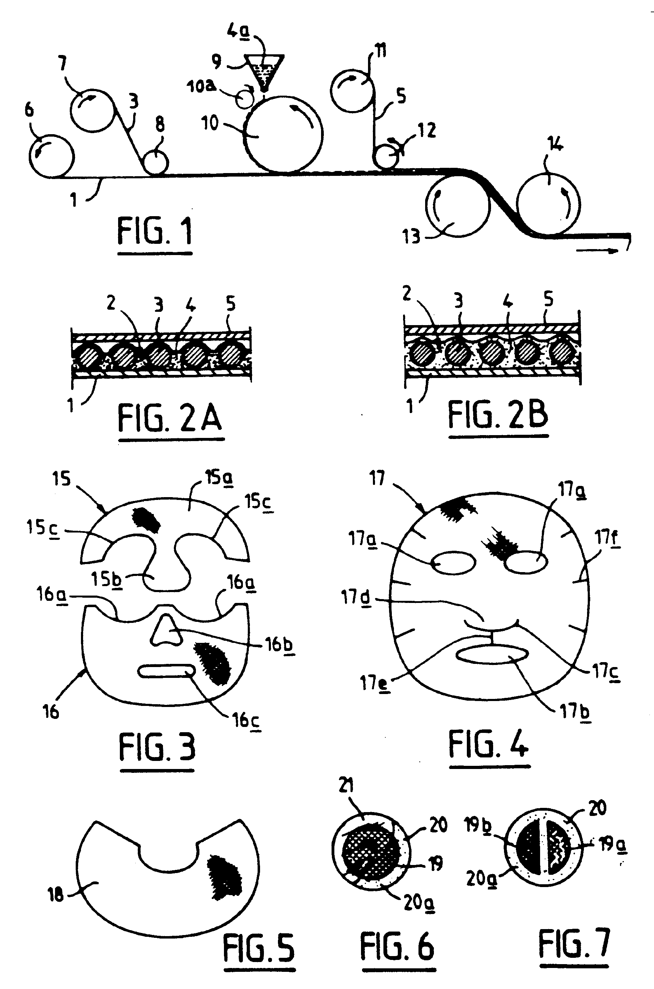

Sur la figure 1, on voit que l'on a schématisé l'appareillage nécessaire pour la fabrication du matériau composite tel que représenté sur les figures 2A et 2B. Ce matériau comporte trois couches superposées, à savoir une feuille-support 1 en polyester, polyéthylène ou papier siliconé, qui permet de glacer la surface de gel à appliquer sur la peau et qui a une bonne tenue en température, un matériau 2 constituant une nappe formée d'un filet 3 en polyamide, entre les mailles duquel est emprisonné un gel 4 au moins partiellement déshydraté, et une feuille supérieure 5 de protection. On déroule une feuille de polyester 1 enroulée sur une bobine 6 et on fait défiler cette feuille horizontalement. On vient alors appliquer sur la feuille 1 ainsi déroulée, une nappe constituée par le filet 3, que l'on déroule de sa bobine 7 et qui passe sous un cylindre 8 disposé juste au-dessus de la feuille 1 pour réaliser l'application du filet 3 sur ladite feuille 1.

En aval du cylindre 8, sur le filet 3 défilant en appui sur la feuille 1, on applique un gel hydraté 4a, à l'état fluide, contenu dans une trémie 9. La trémie 9 comporte, sur son bord inférieur, une fente allongée, par laquelle le gel hydraté 4a s'écoule suivant un rideau qui tombe sur un tambour applicateur 10 ; un deuxième tambour 10a, à distance réglable, sert à l'étalement du gel hydraté 4a ; les tambours 10, 10a ont un axe perpendiculaire au sens d'avancement de la nappe sur son support et le tambour 10 roule sur le filet 3 de façon à y appliquer une couche mince du gel 4a. En aval du tambour applicateur 10, on vient appliquer, à l'aide d'un rouleau 12, une seconde feuille 5, en polyester, qui se déroule à partir d'une bobine 11, sur le filet 3 chargé de gel.

Le matériau composite ainsi formé passe d'abord sur un premier rouleau de calandrage chauffé 13, puis sous un second cylindre de calandrage 14, les feuilles externes 1 et 5 servant à protéger le gel en cours de déshydratation sur le filet, lorsqu'il passe au droit des cylindres 13 et 14.

Suivant l'écartement des tambours 10, 10a, on étale plus ou moins de gel et on obtient un filet 3 plus ou moins chargé en gel, ce qui est représenté sur les figures 2A et 2B, la figure 2B, illustrant le cas où le filet est complètement noyé dans le gel. Le choix concernant la quantité de gel dépend de l'application particulière envisagée.

Après la fabrication, les feuilles 1 et 5 sont retirées si on le souhaite ; il peut être préférable de conserver la feuille 1 pour le conditionnement d'un masque après sont découpage.

En effet, pour les applications, on procède ensuite au découpage du matériau 2 obtenu. Les figures 3 à 5 illustrent différentes possibilités de découpage.

Sur la figure 3, on a prévu un masque pour le visage qui est réalisé en deux parties. La partie supérieure 15 est constituée par une bande principale 15a en forme de couronne, pour venir épouser le front, et elle se prolonge, le long de sa bordure inférieure, dans la région médiane, par une languette 15b qui est destinée à venir recouvrir le nez. Cette languette, qui s'évase vers son extrémité libre, se trouve donc comprise entre deux échancrures 15c qui épousent la forme des arcades sourcilières. La partie restante 16 est destinée à recouvrir le visage, dans la région située sous la ligne moyenne des yeux. A cet effet, elle présente une découpe arrondie à sa partie inférieure pour épouser l'ovale du visage, la bordure supérieure comportant deux échancrures 16a destinées à suivre la ligne inférieure de chaque oeil. Deux ouvertures respectivement 16b et 16c sont également prévues pour le passage respectivement du nez et de la bouche. Ce masque est réalisé en deux parties pour tenir compte de la sphéricité du visage ; de plus , il présente l'avantage d'être réglable en hauteur suivant l'architecture du visage.

Le choix peut également se porter sur un masque du type de celui représenté sur la figure 4. Dans ce cas, il est formé d'une seule pièce, avec deux ouvertures 17a prévues pour les yeux et une ouverture 17b prévue pour la bouche. On prévoit également une échancrure 17c au-dessus de l'ouverture 17b, cette échancrure ménageant un volet 17d, qui est destiné à venir recouvrir le nez. L'échancrure 17c et l'ouverture 17b sont réunies par une fente 17e pour faciliter l'application du masque. De même, on forme, sur chaque bordure latérale, au moins une fente 17f pour réaliser une mise en place plus ajustée de celui-ci, compte tenu de la sphéricité du visage.

Le gel 4 peut contenir des substances actives en mélange avec lui et uniformément réparties au sein du gel. On peut aussi prévoir une application de différents gels renfermant chacun une substance active différente sur un même filet, en des emplacements différents sur le filet, par exemple suivant des traits ou bandes parallèles.

A titre d'exemple, on peut donner quelques précisions concernant un matériau 2 selon l'invention. Le filet 3 peut être un tissu maillé comportant 85 mailles/cm et réalisé avec des fils de polyamide de 150 μm de diamètre ; il est chargé d'un gel d'alcool polyvinylique que l'on étale au niveau du tambour, 10 à 30 % en poids d'extrait sec et que l'on sèche jusqu'à 98 % en poids d'extrait sec. Ce matériau 2 comporte 0,03 g/cm2 de gel après séchage.

On décrira maintenant le mode d'action tout à fait nouveau et original des masques pour le visage qui viennent d'être décrits. L'utilisateur commence par décoller le masque proprement dit, c'est-à-dire le matériau 2, de la feuille-support 1 ; il l'applique sur son visage qu'il a auparavant humidifié, par exemple, à l'aide d'une éponge.

Le masque, alors qu'il est appliqué, perd de sa raideur parce que le gel se réhydrate au contact de la peau humidifiée. Dans ces conditions, le filet retrouve son caractère souple et flexible, immédiatement déformable, lui permettant d'épouser toutes les aspérités du visage.

Le filet adhère alors à la peau, initialement sous forme du contact humide, puis sous forme d'un collage sec. En effet, au bout d'environ 5 à 10 minutes, le gel, réhydraté par l'eau d'humidification de la peau, sèche à l'air, compte tenu de la température propre de la peau. Le gel, toujours prisonnier du filet, devient alors retirable d'un seul tenant, ledit gel ayant , de façon classique, opéré une désincrustation de la peau.

Lorsque le gel renferme au moins une substance traitante, amincissante, anti-rides, ou hydratante, le gel a servi de véhicule pour cette substance au moment où la liaison s'est opérée entre le gel et la peau. Dans ce cas, il peut être souhaitable de garder pendant toute une nuit le masque pour un traitement intra-dermique. Les gels utilisés sont parfaitement compatibles avec la peau et sans danger. Dans le cas où l'on souhaite appliquer également des substances actives non compatibles dans le temps avec le gel et les substances actives qu'il peut contenir, on applique ces substances avec l'eau d'humidification de la peau. On peut éventuellement, dans ce cas, humidifier rapidement par exemple, à l'aide d'un flacon muni d'un tampon, la surface du gel qui sera appliquée sur la peau avec une solution aqueuse ou hydroalcoolique contenant les substances actives non compatibles avec celles contenues dans de gel sec.

La figure 5, montre un élément utilisable dans le raffermissement du buste. Cet élément est en forme de demi-couronne, et son mode d'application et d'action est identique à celui précédemment décrit.

La figure 6 est un schéma illustrant une autre possibilité de l'invention, suivant laquelle le matériau 2 comporte un gel, qui renferme, par exemple, une substance anti-rides ; il est découpé à une forme voulue (par simplification, on a représenté une pastille 19 sur la figure 6), et appliqué sur la couche adhésive 20a revêtant une face d'une feuille-support 20 imperméable à l'eau à l'état liquide ou vapeur, de sorte que la région adhésive soit laissée libre à la périphérie de la feuille 20, en étant protégée, pour le conditionnement, par une feuille détachable 21, par exemple une feuille siliconée.

Pour l'utilisation, la pastille 19 est appliquée avec sa feuillesupport 20, l'adhésif de positionnement 20a faisant occlusion à la périphérie: l'eau déposée sur la peau réhydrate le gel qui libère le cas échéant la substance active ; toutefois, l'eau de transpiration ne s'évapore pas en raison de l'imperméabilité de la feuille 20 et elle est utilisée pour rediluer ladite substance qui peut alors mieux pénétrer dans les pores, qui s'ouvrent davantage par suite de l'occlusion, totale ou partielle suivant la matière choisie pour constituer la feuille 20.

Sur la figure 7 est représentée une variante de l'élément de la figure 6, la pastille 19 étant remplacée par deux pièces 19a, 19b, dont le gel déshydraté renferme des substances actives différentes non compatibles entre elles dans le temps, ces pièces, ayant chacune sensiblement la forme d'un demi-disque, étant donc séparées par un intervalle sur la feuille-support 20. Le mode d'action est identique à celui de l'élément de la figure 6, les substances actives ne pouvant entrer en contact entre elles que lors de leur libération.

**Revendications**

1. Matériau en feuille pour réaliser un traitement cutané local ou un traitement de conditionnement capillaire, lors d'une application temporaire respectivement sur le revêtement cutané ou la chevelure, caractérisé par le fait qu'il est constitué d'un filet flexible (3) tissé ou non tissé, ayant une épaisseur comprise entre 0,01 et 2 mm, comptant environ 2 à 100 mailles par centimètre, le rapport de la surface de ses ouvertures à sa surface totale étant compris entre 5 et 80 %, filet qui constitue l'armature d'une couche de gel hydratable (4) ayant une épaisseur moyenne comprise entre 5 et 1000 μm.

2. Matériau selon la revendication 1, caractérisé par le fait que le filet (3) est réalisé en une matière hydrophobe.

3. Matériau selon les revendications 1 ou 2, caractérisé par le fait que le filet est réalisé en matière transparente.

4. Matériau selon l'une des revendications 1 à 3, caractérisé par le fait que le filet est en substance stérilisable à haute température.

5. Matériau selon l'une des revendications 1 à 4, caractérisé par le fait que le filet est en polyamide.

6. Matériau selon l'une des revendications 1 à 5, caractérisé par le fait que le filet (3) est un filet extensible unidirectionnellement.

7. Matériau selon l'une des revendications 1 à 6, caractérisé par le fait que le gel (4) est constitué d'au moins un polymère hydrosoluble ou hydrogonflable, présentant un extrait sec compris entre 0,5 et 50 % en poids.

8. Matériau selon l'une des revendications 1 à 7, caractérisé par le fait que les polymères constituant le gel sont choisis dans le groupe formé par l'alcool polyvinylique, les sels de métaux alcalins de carboxyméthylcellulose réticulée, les sels de métaux alcalins d'acide polyacrylique, les poly(oxyde d'alkylène) réticulés, les sels de métaux alcalins de polymères greffés cellulose-acrylonitrile ou amidonacrylonitrile contenant des groupements carboxyliques, la gomme adragante, la gomme arabique, la gomme de guar et ses dérivés, les alginates, la gomme de xanthane, les autres dérivés de la cellulose, l'albumine, la gélatine, le galactomannane, le polyacrylamide.

9. Matériau selon l'une des revendications 1 à 8, caractérisé par le fait que le gel (4) renferme au moins une substance active de traitement, choisie notamment dans le groupe formé par les actifs hydrosolubles, l'argile, le kaolin, la silice, les huiles émulsionnées, les agents hydratants, amincissants, anti-rides, stimulants, revitalissants, raffermissants et adoucissants, les poudres.

10. Matériau selon la revendication 9, caractérisé par le fait que le gel (4) renferme au moins deux substances actives, lesdites substances étant localisées en des emplacements différents du filet (3).

11. Matériau selon l'une des revendications 1 à 10, caractérisé par le fait que le filet (3), imprégné par le gel (4) est accolé, sur au moins une partie de l'une de ses faces, à une feuille-support (1).

12. Matériau selon la revendication 11, caractérisé par le fait que chacune de ses deux faces est associée à une feuille-support protectrice détachable (1,5).

13. Matériau selon la revendication 12, caractérisé par le fait que la feuille-support (1) est, au moins partiellement, imperméable à l'eau à l'état liquide ou vapeur.

14. Article réalisé au moyen du matériau selon l'une des revendications 1 à 13, caractérisé par le fait qu'il présente une découpe adaptée à la forme de la partie du corps à traiter.

15. Article selon la revendication 14, destiné au traitement du visage, caractérisé par le fait qu'il est réalisé en deux parties, l'une (15) correspondant au front et se prolongeant le long de sa bordure inférieure, entre deux échancrures (15c) qui suivent le contour des arcades sourcillières, par une patte (15b) destinée à recouvrir le nez et l'autre (16), correspondant à la région du visage se situant au-dessous des yeux et comportant deux ouvertures (16b, 16c) correspondant respectivement à la bouche et au nez, la bordure supérieure de cette partie (16) du masque comportant deux échancrures (16a) pour suivre la ligne inférieure des yeux.

16. Article selon la revendication 14, destiné au traitement du visage, caractérisé par le fait qu'il est constitué d'une seule pièce (17) découpée à la dimension et à la forme du visage, ladite pièce (17) comprenant trois ouvertures (17a, 17a, 17b) correspondant respectivement aux yeux et à la bouche, ainsi qu'une découpe (17c) correspondant à la région médiane et inférieure du nez et dégageant un volet (17d) destiné à venir recouvrir le nez, une fente médiane (17e) reliant l'ouverture (17b) correspondant à la bouche et ladite découpe (17c), des fentes transversales (17f) étant éventuellement pratiquées le long des bordures latérales de ladite pièce (17).

17. Article selon la revendication 14, destiné au traitement du buste, caractérisé par le fait qu'il présente une découpe sensiblement en forme de demi-couronne.

18. Matériau selon la revendication 11, caractérisé par le fait que le filet (3) imprégné par le gel (4) est disposé sur le côté adhésif d'une feuille-support (20) revêtue sur l'une de ses faces, d'une couche d'adhésif (20a), ladite feuille (20) dépassant dudit filet (3) sur tout son pourtour, une pellicule détachable (21) venant protéger au moins la partie adhésive (20a) avant emploi.

19. Matériau selon l'une des revendications 1 à 11 et 18, caractérisé par le fait qu'il est associé à un matériau spongieux saturé d'au moins une substance de traitement non compatible dans le temps avec le gel (4) et/ou avec la (ou les) substance(s) de traitement contenue(s) le cas échéant dans le gel.

20. Procédé de fabrication d'un matériau selon la revendication 11, caractérisé par le fait que :

– on place une nappe constituant un filet flexible (3) sur une feuille-support (1), capable de résister à la température de chauffage du gel hydraté (4a) à l'étape ultérieure de séchage ;

– on dépose sur ladite nappe ainsi supportée, une mince couche de gel hydraté (4a) se trouvant dans un état liquide ou extrudable et renfermant, le cas échéant, au moins une substance active de traitement, de façon à charger les mailles du filet (3) avec ledit gel hydraté (4a) et

– on sèche ledit gel hydraté (4a) jusqu'à atteindre le degré de déshydratation désiré.

21. Procédé selon la revendication 20, caractérisé par le fait que l'on réalise le dépôt de la couche de gel hydraté (4a) par calandrage ou par extrusion.

22. Procédé selon l'une des revendications 20 ou 21, caractérisé par le fait que l'on réalise le séchage du gel hydraté (4a) prisonnier du filet par calandrage ou par passage du filet (3) dans un tunnel infra-rouge ou par soufflage d'air chaud sur le filet (3), une pellicule de protection (5) étant placée par-dessus ledit filet (3) dans le cas où l'on procède par calandrage.


**Patentansprüche**

1. Blattförmiges Material zur lokalen Hautbehandlung oder Haarkonditionierungsbehandlung, welches vorübergehend auf die Haut oder die Haare gelegt wird, **dadurch gekennzeichnet** , daß es aus einem flexiblen, gegebenenfalls gewebten Netz (3) besteht, welches eine Dicke von 0,01-2 mm aufweist und etwa 2-100 Maschen/cm umfaßt, wobei das Verhältnis der Oberfläche seiner Öffnungen zu seiner Gesamtoberfläche 5-80 % beträgt und das Netz eine Verstärkung einer dünnen hydratisierbaren Gelschicht (4) mit einer mittleren Dichte von 5-1000 µm darstellt.

2. Material nach Anspruch 1, **dadurch gekennzeichnet** , daß das Netz (3) aus einem hydrophoben Material gefertigt ist.

3. Material nach den Ansprüchen 1 oder 2, **dadurch gekennzeichnet** , daß das Netz aus einem transparenten Material gefertigt ist.

4. Material nach einem der Ansprüche 1 bis 3, **dadurch gekennzeichnet** , daß das Netz im wesentlichen bei hoher Temperatur sterilisierbar ist.

5. Material nach einem der Ansprüche 1 bis 4, **dadurch gekennzeichnet** , daß das Netz aus einem Polyamid besteht.

6. Material nach einem der Ansprüche 1 bis 5, **dadurch gekennzeichnet** , daß das Netz (3) ein nur in einer Richtung dehnbares Netz ist.

7. Material nach einem der Ansprüche 1 bis 6, **dadurch gekennzeichnet**, daß das Gel (4) aus mindestens einem wasserlöslichen oder in Wasser quellbaren Polymer besteht, welches als Trockenextrakt 0,5 bis 50 Gew.-% ausmacht.

8. Material nach einem der Ansprüche 1 bis 7, **dadurch gekennzeichnet** , daß die Polymere, aus welchen das Gel besteht, ausgewählt sind unter Polyvinylalkohol, den Alkalimetallsalzen von vernetzter Carboxymethylcellulose, den Alkalimetallsalzen von Polyacrylsäure, vernetzten Polyalkylenoxiden, den Alkalimetallsalzen von Polymeren, die mit Celluloseacrylnitril oder Stärkeacrylnitril gepfropft sind und Carboxylgruppen enthalten, Tragantgummi, Gummi arabicum, Guargummi und Derivaten davon, Alginaten, Xanthangummi, anderen Cellulosederivaten, Albumin, Gelatine, Galactomannan und Polyacrylamid.

9. Material nach einem der Ansprüche 1 bis 8, **dadurch gekennzeichnet** , daß das Gel (4) mindestens einen Behandlungswirkstoff enthält, insbesondere ausgewählt unter wasserlöslichen Wirkstoffen, Ton, Kaolin, Kieselerde, emulgierten Ölen, Hydratisierungsmitteln, Verdünnungsmitteln, Antifaltenmitteln, Stimulanzien, Revitalisierungsmitteln, Straffungsmitteln, weichmachenden Mitteln und Pudern.

10. Material nach Anspruch 9, **dadurch gekennzeichnet** , daß das Gel (4) mindestens zwei Wirkstoffe enthält, wobei diese Wirkstoffe sich an verschiedenen Stellen des Netzes (3) befinden.

11. Material nach einem der Ansprüche 1 bis 10, **dadurch gekennzeichnet** , daß das Netz (3), welches mit dem Gel (4) imprägniert ist, in mindestens einem Abschnitt seiner Außenseiten an eine Trägerfolie (1) gebunden ist.

12. Material nach Anspruch 11, **dadurch gekennzeichnet** , daß jede der zwei Außenseiten an eine schützende abnehmbare Trägerfolie (1, 5) gebunden ist.

13. Material nach Anspruch 12, **dadurch gekennzeichnet** , daß die Trägerfolie (1) mindestens teilweise für Wasser in flüssigem Zustand oder für Dampf undurchlässig ist.

14. Gegenstand, gefertigt aus dem Material nach einem der Ansprüche 1 bis 13, **dadurch gekennzeichnet** , daß er als Zuschnitt vorliegt, der der Form eines zu behandelnden Körperteils angepaßt ist.

15. Gegenstand nach Anspruch 14 zur Behandlung des Gesichts, **dadurch gekennzeichnet** , daß er aus zwei Teilen besteht, wobei der eine Teil (15) der Stirn entspricht und sich an seiner Innenkante zwischen zwei bogenförmigen Ausschnitten (15c), die den Konturen der Augenbrauenbögen folgen, in einer Lasche (15b) zur Abdeckung der Nase fortsetzt, und der andere Teil (16) dem unterhalb der Augen liegenden Gesichtsbereich entspricht, und zwei Öffnungen (16b, 16c) aufweist, die Mund bzw. Nase entsprechen, wobei der obere Rand dieses Maskenteils (16) zwei bogenförmige Ausschnitte (16a) aufweist, die der unteren Augenlinie folgen.

16. Gegenstand nach Anspruch 14 zur Behandlung des Gesichts, **dadurch gekennzeichnet** , daß er aus einem einzigen Stück (17) besteht, welches der Gesichtsgröße und -form entsprechend zugeschnitten ist, wobei das Stück (17) drei Öffnungen (17a, 17a, 17b) hat, die den Augen und dem Mund entsprechen sowie einen Einschnitt (17c) hat, der dem Bereich mitten unterhalb der Nase entspricht und eine Klappe (17d) zum Bedecken der Nase öffnet, daß er einen mittigen Schlitz (17e), der die dem Mund entsprechende,Öffnung (17b) und den Einschnitt (17c) verbindet, und transversale Schlitze (17f), die gegebenenfalls entlang den seitlichen Rändern.des Stückes (17) ausgebildet sind, aufweist.

17. Gegenstand nach Anspruch 14 zur Behandlung der Brust, dadurch **gekennzeichnet** , daß er einen im wesentlichen halbkranzförmigen Zuschnitt hat.

18. Material nach Anspruch 11, **dadurch gekennzeichnet**, daß das mit Gel (4) imprägnierte Netz (3) auf der Klebeseite einer Trägerfolie (20) angebracht ist, die auf einer ihrer Seiten mit einer Klebeschicht (20a) bedeckt ist, wobei die Folie (20) das Netz (3) auf seinem ganzen Umfang überragt und ein abziehbarer Film (21) zumindest den Klebeteil (20a) vor Gebrauch schützt.

19. Material nach einem der Ansprüche 1 bis 11 und 18, **dadurch gekennzeichnet**, daß es mit einem Schaumstoff verbunden ist, der mit mindestens einer Behandlungssubstanz gesättigt ist, die mit dem Gel (4) und/ oder mit der (oder den) Behandlungssubstanz(en), die gegebenenfalls im Gel enthalten ist (sind), auf Dauer nicht verträglich ist (sind).

20. Verfahren zur Herstellung eines Materials nach Anspruch 11, **dadurch gekennzeichnet**, daß man

– ein aus einem flexiblen Netz (3) bestehendes Gewebe auf eine Trägerfolie (1) aufbringt, die bei der Temperatur der Erwärmung des hydratisierten Gels (4a) in der letzten Phase der Trocknung beständig ist;

– auf das auf diese Weise verstärkte Gewebe eine dünne Schicht hydratisierten Gels (4a) aufträgt, welches in einem'flüssigen oder spritzfähigen Zustand ist und gegebenenfalls mindestens einen Behandlungswirkstoff enthält, wobei die Maschen des Netzes (3) mit dem hydratisierten Gel (4a) beladen werden; und

– das hydratisierte Gel (4a) solange trocknet, bis man den gewünschten Dehydratisierungsgrad erreicht hat.

21. Verfahren nach Anspruch 20, **dadurch gekennzeichnet**, daß die Schicht des hydratisierten Gels (4a) durch Kalandrieren oder Extrudieren aufgetragen wird.

22. Verfahren nach einem der Ansprüche 20 oder 21, **dadurch gekennzeichnet**, daß die Trocknung des im Netz eingeschlossenen hydratisierten Gels (4a) durch Kalandrieren erfolgt oder das Netz (3) in einen Infrarot-Tunnel gegeben oder Heißluft auf das Netz (3) geblasen wird, wobei ein Schutzfilm (5) über das Netz (3) gelegt wird, wenn kalandriert wird.

**Claims**

1. A sheet material for effecting a local skin treatment or a hair conditioning treatment during a temporary application to the cutaneous covering or the scalp respectively, characterized in that it is constituted by a flexible netting (3), woven or non-woven, having a thickness comprised between 0.01 and 2 mm, counting approximately 2 to 100 meshes per centimetre, the ratio of the surface of its openings to its total surface being comprised between 5 and 80%, which netting constitutes the reinforcement for a hydratable gel layer (4) having a mean tickeness comprised between 5 and 1000 μm.

2. A material according to claim 1, characterized in that the netting (3) is made of a hydrophobic material.

3. A material according to claims 1 or 2, characterized in that the netting is made of a transparent material.

4. A material according to one of claims 1 to 3, characterized in that the netting is made of a substance sterilizable at a high temperature.

5. A material according to one of claims 1 to 4, characterized in that the netting is made of polyamide.

6. A material according to one of claims 1 to 5, characterized in that the netting (3) is a unidirectionally extensible netting.

7. A material according to one of claims 1 to 6, characterized in that the gel (4) is constituted by at least one hydrosoluble polymer or one capable of swelling under the effect of water, having a dry extract comprised between 0.5 and 50% by weight.

8. A material according to one of claims 1 to 7, characterized in that the polymers constituting the gel are chosen form the group formed by polyvinyl alcohol, the salts of alkaline metals of reticulated carboxylmethyl cellulose, the salts of alkaline metals of polyacrylic acid, reticulated poly-(alkylene oxides) the salts of alkaline metals of acrylonitrile-cellulose grafted polymers or acrylonitrile starch grafted polymers containing carboxylic groups, gum tragacanth, gum arablic, guar gum and its derivatives, the alginates, xanthane gum the other cellulose derivatives, albumin, gelatine, galactomannane, polyacrylamide.

9. A material according to one of claims 1 to 8, characterized in that the gel (4) contains at least one active treatment substance, chosen in particular from the group formed by water soluble active substances, clay, kaolin, silica, emulsified oils, hydrating agents, thinning agents, anti-wrinkle agents, stimulants, revitalising agents, firming and softening agents, powders.

10. A material according to claim 9, characterized in that the gel (4) contains at least two active substances, the said substances being disposed at different locations of the netting (3).

11. A material according to one of claims 1 to 10, characterized in that the netting (3) impregnated by the gel (4) is attached, over at least a portion of one of its sides, to a backing sheet (1).

12. A material according to claim 11, characterized in that each one of its two sides is attached to a detachable protective backing sheet (1, 5).

13. A material according to claim 12, characterized in that the backing sheet (1) is at least partly impermeable to water in its liquid or vapour state.

14. An article made by means of a material according to one of claims 1 to 13, characterized in that it has a cutout adapted to the shape of the part of the body to be treated.

15. An article according to claim 14 intended for the treatment of the face, characterized in that it is made in two parts, the one (15) corresponding to the forehead and being extended along its bottom edge between two cutouts (15c) which follow the contour of the eyebrow arches, by a tab (15b) intended to cover the nose, and the other, (16) corresponding to the region of the face situated below the eyes and comprising two openings (16b, 16c) corresponding respectively to the mouth and nose, the upper edge of this part (16) of the mask comprising two cutouts (16a) to follow the lower line of the eyes.

16. An article according to claim 14 intended for facial treatment, characterized in that it is formed by a single piece (17) cut to the size and shape of the face, the said piece (17) comprising three openings (17a, 17a, 17b) corresponding respectively to the eyes and the mouth, as well as a cutout (17c) corresponding to the median and lower region of the nose and freeing a flap (17d) intended to come to cover the nose, a median slit (17e) joining the opening (17b) corresponding to the mouth and the said cutout (17c), transverse slits (17f) being optionally cut along the lateral edges of the said piece (17).

17. An article according to claim 14 intended for the treatment of the bust, characterized in that it has a cutout substantially in a half-wreath shape.

18. A material according to claim 11, characterized in that the netting (3) impregnated by the gel (4) is disposed on the adhesive side of a backing sheet (20) coated on one of its sides with an adhesive layer (20a), the saidj sheet (20) projecting over the said netting (3) over ist whole periphery, a detachable film (21) coming to protect at least the adhesive portions (20a) before use.

19. A material according to one of claims 1 to 11 and 18, characterized in that it is associated with a spongy material saturated with at least one treatment substance not compatible over a period of time with the gel (4) and/or with the treatment substance or substances contained in the gel if required.

20. A method for manufacturing a material according to claim 11, characterized in that:
– a cloth constituting a flexible netting (3) is placed on a backing sheet (1) capable of withstanding the heating temperature of the hydrated gel (4a) in the subsequent drying stage;
– on the said cloth thus backed, there is deposited a thin hydrated gel layer (4a) in a liquid or extrudable state and containing, if required, at least one active treatment substance so as to charge the meshes of the netting (3) with the said hydrated gel (4a); and
– the said hydrated gel (4a) is dried until the desired degree of dehydration is reached.

21. A method according to claim 20, characterized in that the hydrated gel layer 4a is deposited by calendering or by extrusion.

22. A method according to one of claims 20 or 21, characterized in that the drying of the hydrated gel layer (4a) trapped in the netting is effected by calendering or by causing the netting (3) to pass into an infra-red tunnel or by blowing hot air over the netting (3), a protective film (5) being placed above the netting (3) in the case where the calendering method is adopted.

FIG. 1

FIG. 2A

FIG. 2B

FIG. 3

FIG. 4

FIG. 5

FIG. 6

FIG. 7

11